# EUROPÄISCHE PATENTANMELDUNG

(11) **EP 1 212 978 A2**
(43) Veröffentlichungstag der Anmeldung: **12.06.2002**
(21) Anmeldenummer: 01128778.6
(22) Anmeldetag: 03.12.2001
(51) Int. Cl.: A61B 5/00

(54) **Verfahren zur Bestimmung der Konzentration einer Substanz in biologischen Flüssigkeiten**

(30) Priorität: 06.12.2000 DE 10060589
(71) Anmelder: Institut für Diabetestechnologie gemeinnützige Forschungs- und Entwicklungsgesellschaft mbH, 89081 Ulm (DE)
(72) Erfinder: Pfeiffer, Boris, 89081 Ulm (DE)
(74) Vertreter: Dziewior, Joachim, Dipl.-Phys. Dr.

(57) **Zusammenfassung**

Verfahren zur Bestimmung der Konzentration einer Substanz in der Gewebeflüssigkeit biologischer Organismen.

Das Verfahren dient zur Bestimmung der Konzentration einer Substanz in der Gewebeflüssigkeit biologischer Organismen, insbesondere von Substanzen wie Glukose, Laktat und dergleichen. Dazu wird einer im Gewebe implantierten Mikrodialysesonde ein Perfusatstrom zugeführt und nach Anreicherung mit der in der Gewebeflüssigkeit enthaltenen Substanz als Dialysatstrom entnommen. Die Substanz wird unter Einwirkung eines Enzyms oxidiert, wobei der zur Oxidation der Substanz erforderliche Sauerstoff über ein Lösungsmittel beigefügt wird, das eine hohe Aufnahmefähigkeit für Gase in physikalisch gelöster Form aufweist.

## Beschreibung

Die Erfindung betrifft ein Verfahren zur Bestimmung der Konzentration einer Substanz in biologischen Flüssigkeiten, insbesondere von Substanzen wie Glukose, Laktat und dergleichen, bei welchem der biologischen Flüssigkeit wenigstens die zu bestimmende Substanz entnommen und unter Einwirkung eines Enzyms oxidiert wird.

Ein derartiges Verfahren ist aus der DE 41 30 742 bekannt. Um die kontinuierliche Bestimmung der zu überwachenden Substanz, also beispielsweise Glukose oder Milchsäure, zu ermöglichen, erfolgt eine Probenentnahme aus dem Körpergewebe nach dem Dialyseverfahren. Dazu ist eine im Körpergewebe implantierbare Dialysesonde vorgesehen, die die Gestalt eines doppellumigen Katheders aufweist. Diese Sonde weist eine geschlossene Dialysemembran sowie einen Zuflußkanal für den Perfusatstrom und einen Rückflußkanal für den Dialysatstrom auf. Der Rückflußkanal führt zu einer Messanordnung, die eine elektrochemische Enzymzelle aufweist, die eine driftfreie Konzentrationsmessung über eine möglichst lange Messdauer ermöglichen soll.

Bei diesem Meßverfahren zeigte sich, daß durch einen allmählichen Verlust an Enzymaktivität eine Meßsignaldrift auftrat. Dies macht einen regelmäßigen Austausch des enzymbeschichteten Sensors erforderlich, was im Hinblick auf die Herstellungs- und Materialkosten mit nicht unerheblichen Aufwand verbunden ist. Daher wurde dieses Verfahren gemäß der DE 44 01 400 dahingehend verbessert, daß an einer Mischstelle dem Dialysatstrom kontinuierlich ein Enzym zugemischt und der so gebildete Meßdialysatstrom einem elektrochemischen Meßsensor zugeführt wird. Dieser bestimmt die Konzentration der Substanz unter selektiver Einwirkung des Enzyms auf amperometrischem Wege.

Wichtig für dieses Meßverfahren ist ein vollständiger Umsatz der im Dialysatstrom enthaltenen, zu messenden Substanz. Handelt es sich bei der Substanz um Glukose, so läuft die Reaktion derart ab, daß die Glukose mit Sauerstoff zu Gluconolacton und Wasserstoffperoxid umgesetzt wird. Letzteres läßt sich dann amperometrisch bestimmen. Als Katalysator für diese Reaktion dient beispielsweise Glukoseoxidase (GOD). Der für die Reaktion erforderliche Sauerstoff löst sich jedoch schlecht in wässrigen Medien.

Um die Linearität der Messung auch bei höheren Konzentrationen der zu bestimmenden Substanz zu gewährleisten, muß daher der für die Oxidation benötigte Sauerstoff in ausreichender Konzentration vorliegen. Dazu wird der Meßdialysatstrom vor der Messung einer Sauerstoff-Atmosphäre ausgesetzt, was beispielsweise über einen Sauerstoff-permeablen Reaktionsschlauch erfolgen kann. Dieser Reaktionsschlauch kann beispielsweise aus Silikon bestehen, da Silikon für Sauerstoff ausreichend durchlässig ist.

Allerdings hat sich gezeigt, daß z. B. bei der komparativen Gewebeglukosemessung bis zum vollständigen Umsatz mindestens 12 cm Silikonschlauch benötigt werden, der dann ein Innenvolumen von etwa 8 Mikroliter aufweist. Das Meßdialysat wird dadurch beim Transport durch den "Sauerstoffschlauch" verdünnt, was die Auswertung der Einzelmessungen erschwert.

Der Erfindung liegt die Aufgabe zugrunde, das Verfahren der eingangs genannten Art so zu verbessern, daß auch ohne die Anwendung eines "Sauerstoffschlauches" eine vollständige Umsetzung des Reaktionsgemisches durch eine ausreichend hohe Sauerstoffkonzentration gewährleistet ist.

Diese Aufgabe wird nach der Erfindung dadurch gelöst, daß der zur Oxidation der Substanz erforderliche Sauerstoff über ein Lösungsmittel beigefügt wird, das eine hohe Aufnahmefähigkeit für Gase in physikalisch gelöster Form aufweist.

Der durch die Erfindung erreichte Vorteil besteht im wesentlichen darin, daß durch die Beigabe des Sauerstoffs über ein Lösungsmittel zum Dialysatstrom dieser für die erforderliche Reaktion in ausreichender Konzentration zur Verfügung steht. Hierbei ist weiter vorteilhaft, daß z. B. durch Kavitation und andere Effekte entstehende und u.U. störend wirkende Gasblasen (die insbesondere aus Stickstoff und/oder aus Sauerstoff bestehen können) infolge der guten Löslichkeit in dem Lösungsmittel von diesem aufgenommen und somit aus dem Mikrofluidiksystem schnell entfernt werden.

In bevorzugter Ausgestaltung der Erfindung ist zur Entnahme der Substanz aus einem Organismus eine im Gewebe implantierte Mikrodialysesonde vorgesehen, der ein Perfusatstrom zugeführt und nach Anreicherung mit der in der Gewebeflüssigkeit enthaltenen Substanz als Dialysatstrom entnommen wird.

Grundsätzlich besteht selbstverständlich auch die Möglichkeit, die Gewinnung der benötigten biologischen Flüssigkeit auf eine andere Weise vorzunehmen. Hierzu bietet sich die Ultrafiltration gemäß US-Patent 4 832 034 oder auch die open flow Microperfusion (vgl. L. Schaupp et al., Am.J.Physiol.Endocrinol.Metab. 276,E401-E408,1999) an. Ebenso ist der Einsatz transdermaler Sensoren denkbar, wobei die Gewinnung der Gewebeflüssigkeit entweder durch reverse Iontopherese (vgl. L. Heinemann, Diabetes und Stoffwechsel 9 (2000) S. 167 ff) oder ebenfalls auf transkutanem Wege durch Mikrotraumatisierung (siehe einschlägiges Datenblatt der Firma Rösch AG Medizintechnik, 12349 Berlin) erfolgen kann.

Als besonders geeignet im Rahmen der Erfindung hat sich erwiesen, wenn als Lösungsmittel ein Fluorkohlenstoff oder eine perfluorierte Verbindung vorgesehen ist. Dabei kann es sich um teilfluorierte oder perfluorierte Kohlenwasserstoffe bzw. um perfluorierte Kohlenwasserstoffe mit funktionellen Gruppen handeln. Die Löslichkeit von Sauerstoff in Fluorkohlenstoff oder einer perfluorierten Verbindung ist üblicherweise etwa 20 bis 30 mal größer als in Wasser, wobei jedoch nur solche Lösungsmittel in Betracht kommen, in denen die Löslichkeit von Sauerstoff jedenfalls größer als in Wasser ist.

Da jedoch Fluorkohlenstoffe oder eine perfluorierte Verbindung selbst in Wasser in der Regel nicht lösbar sind, wird im Rahmen der Erfindung vorgeschlagen, daß der den Sauerstoff tragende Fluorkohlenstoff oder die perfluorierte Verbindung in Form einer Emulsion aufbereitet sind.

Schließlich hat es sich als vorteilhaft herausgestellt, wenn der den Sauerstoff tragende Fluorkohlenstoff oder die perfluorierte Verbindung gemeinsam mit dem Enzym dem Dialysatstrom und/oder dem Perfusatstrom zugeführt wird.

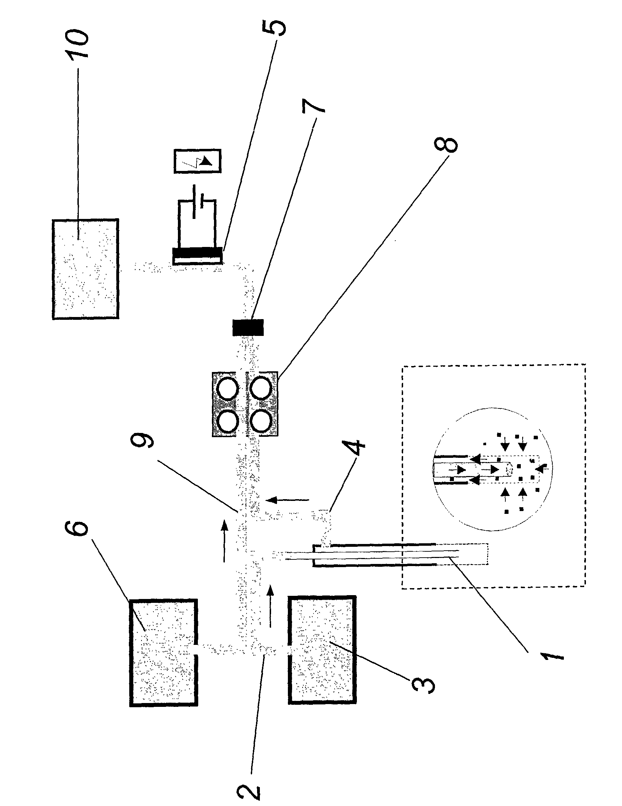

Im folgenden wird die Erfindung an einem in der Zeichnung dargestellten Ausführungsbeispiel näher erläutert; die einzige Figur zeigt einen zur Durchführung des Verfahrens geeigneten Aufbau in schematischer Darstellung.

Die Anordnung zur Überwachung der Konzentration bestimmter Substanzen, beispielsweise Glukose oder Laktat, im Körpergewebe besteht im wesentlichen aus einer in das Subkutangewebe eines Patienten implantierbaren Mikrodialysesonde 1 und einer extrakorporal angeordneten Meßanordnung. Die Mikrodialysesonde 1 wird eingangsseitig über eine Perfusatleitung 2 mit einem Perfusatstrom aus einem Perfusatreservoir 3 beaufschlagt und steht ausgangsseitig über eine Dialysatleitung 4 mit einem Meßsensor 5 in Verbindung, der in hier nicht näher zu erläuternder Weise die Konzentration der zu prüfenden Substanz ermittelt. Die als Katalysator wirkende Enzymlösung wird in einem Enzymlösungsreservoir 6 bereitgestellt und über ein T-Stück 7 dem Dialysatstrom beigegeben.

Für die Förderung der Flüssigkeiten ist eine Rollendosierpumpe 8 vorgesehen, die sowohl auf die Perfusatleitung 2 als auch auf die Leitung 9 der Enzymlösung wirkt. In Strömungsrichtung hinter dem Meßsensor 5 ist ein Abfallbehältnis 10 vorgesehen, das das Meßdialysat nach der Auswertung durch den Meßsensor 5 aufnimmt.

Bei der Anwendung dieser Anordnung wird der in die Mikrodialysesonde 1 fließende Perfusatstrom mit der in der Gewebeflüssigkeit enthaltenen Substanz angereichert und sodann der Microdialysesonde 1 als Dialysatstrom entnommen. Die Substanz wird anschließend unter Einwirkung des beigegebenen Enzyms oxidiert, wobei der zur Oxidation der Substanz erforderliche Sauerstoff über ein Lösungsmittel beigefügt wird. Dieses Lösungsmittel weist eine hohe Aufnahmefähigkeit für Gase in physikalisch gelöster Form auf, wofür sich insbesondere Fluorkohlenstoffe oder eine perfluorierte Verbindung eignen. Wegen ihrer geringen Löslichkeit in Wasser werden sie in Form einer Emulsion beigegeben, wobei es sich insbesondere anbietet, diese Emulsion mit in das Enzymlösungsreservoir 6 einzufüllen.

## Patentansprüche

1. Verfahren zur Bestimmung der Konzentration einer Substanz in biologischen Flüssigkeiten, insbesondere von Substanzen wie Glukose, Laktat und dergleichen, bei welchem der biologischen Flüssigkeit wenigstens die zu bestimmende Substanz entnommen und unter Einwirkung eines Enzyms oxidiert wird, **dadurch gekennzeichnet, daß** der zur Oxidation der Substanz erforderliche Sauerstoff über ein Lösungsmittel beigefügt wird, das eine hohe Aufnahmefähigkeit für Gase in physikalisch gelöster Form aufweist.

2. Verfahren nach Anspruch 1, **dadurch gekennzeichnet, daß** zur Entnahme der Substanz aus einem Organismus eine im Gewebe implantierte Mikrodialysesonde (1) vorgesehen ist, der ein Perfusatstrom zugeführt und nach Anreicherung mit der in der Gewebeflüssigkeit enthaltenen Substanz als Dialysatstrom entnommen wird.

3. Verfahren nach Anspruch 1 oder 2, **dadurch gekennzeichnet, daß** als Lösungsmittel ein Fluorkohlenstoff oder eine perfluorierte Verbindung vorgesehen ist.

4. Verfahren nach Anspruch 3, **dadurch gekennzeichnet, daß** der den Sauerstoff tragende Fluorkohlenstoff oder die perfluorierte Verbindung in Form einer Emulsion aufbereitet sind.

5. Verfahren nach Anspruch 3 oder 4, **dadurch gekennzeichnet, daß** das den Sauerstoff tragende Fluorkohlenstoff oder eine perfluorierte Verbindung gemeinsam mit dem Enzym dem Dialysatstrom und/oder dem Perfusatstrom zugeführt wird.
